# EUROPÄISCHE PATENTSCHRIFT

(11) **EP 1 013 630 B1**
(45) Veröffentlichungstag und Bekanntmachung des Hinweises auf die Patenterteilung: **27.03.2002**
(21) Anmeldenummer: 99123396.6
(22) Anmeldetag: 24.11.1999
(51) Int. Cl.: C07C 17/25, C07C 21/06

(54) **Verfahren zur Verdampfung von 1,2-Dichlorethan (EDC)**
Process for the evaporation of 1,2-dichloroethane (EDC)
Procédé pour l' évaporation de 1,2-dichloroéthane (EDC)

(30) Priorität: 22.12.1998 DE 19859262
(43) Veröffentlichungstag der Anmeldung: 28.06.2000
(73) Patentinhaber: Krupp Uhde GmbH, 44141 Dortmund (DE)
(72) Erfinder: Seidelbach, Friedrich, 65207 Wiesbaden (DE)
(74) Vertreter: Patentanwälte Meinke, Dabringhaus und Partner

(56) Entgegenhaltungen:
- EP-A- 0 276 775
- GB-A- 740 536
- US-A- 4 798 914

## Beschreibung

Die Erfindung richtet sich auf ein Verfahren zur Verdampfung von 1,2-Dichlorethan (EDC) vor der thermischen Zersetzung (Pyrolyse).

Die Herstellung von Vinylchlorid (VCM) erfolgt technisch in der Hauptsache durch nicht katalytische und unvollständige thermische Zersetzung (Pyrolyse) von gasförmigem 1,2-Dichlorethan (EDC), wobei als Koppelprodukt Chlorwasserstoff (HCL) gebildet wird, z.B. GB-A-740 536 und US-A-4 798 914.

Die technisch gebräuchlichen, nicht katalytischen Verfahren, z.B. EP-0 276 775-B1, werden unter einem Druck von 1 bis 3 MPa und bei einer Austrittstemperatur aus der Pyrolyse-Zone von 450°C bis 600°C ausgeführt. Die Eintrittstemperaturen in die Pyrolyse-Zone liegen dabei bei 200°C bis 300°C.

Aus der US-A 5 488 190 ist ein Verfahren zur Vermeidung von Nebenprodukten bei der Pyrolyse von EDC bekannt, wobei dort die Wärmeübertragung durch eine Rohrwand im Direktkontakt mit einem dem EDC zugemischten und hochaufgeheizten Gas oder Feststoff erfolgt. Die EDC-Verdampfung erfolgt dabei nur im unterkritischen Druck- und Temperaturbereich.

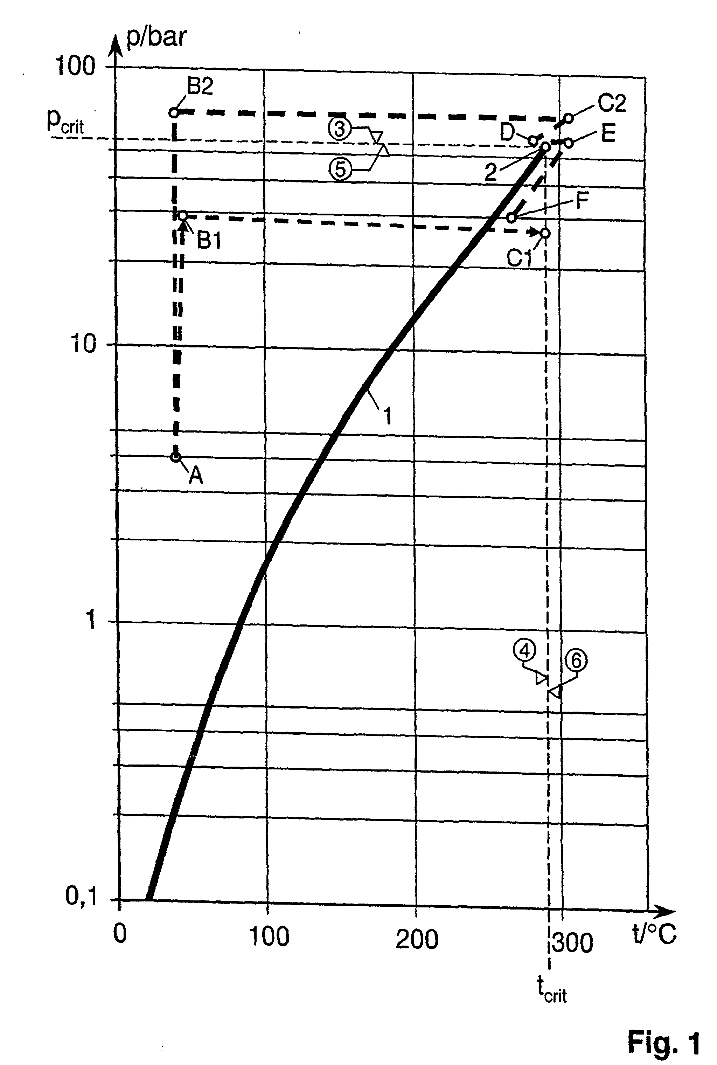

Die DE-A 35 43 222 beschreibt ein verbessertes Verfahren zur Herstellung von Vinylchlorid durch thermische Spaltung von 1,2-Dichlorethan vom flüssigen zum gasförmigen EDC im unterkritischen Bereich, wie sich aus der dortigen Fig. 1 ergibt.

Die Pyrolyse-Zone wird bei den technischen Verfahren als mit Erdgas oder Heizöl beheizter Ofen ausgeführt, in dessen Feuerungsraum sich eine oder mehrere Rohrschlangen befinden, in deren Inneren die EDC-Pyrolyse stattfindet.

Die Verweilzeit in der Pyrolyse-Zone und die Temperatur werden so gewählt, daß einerseits möglichst wenig unerwünschte Nebenprodukte, wie Ruß (Koks), chlorierte und ungesättigte Kohlenwasserstoffe sowie Benzol, gebildet werden und andererseits aus wirtschaftlichen Gründen der EDC-Umsatz bei mindestens 50 % liegt.

Die Ursache der Rußbildung ist bei den üblichen technischen Verfahren keineswegs nur allein auf die relativ hohen Temperaturen in der Pyrolyse zurückzuführen. Vor der eigentlichen Pyrolyse wird das üblicherweise flüssig vorliegende EDC verdampft und eventuell überhitzt.

Verdampfer weisen (wie z.B. auch Siedereaktoren) ein für zur Zersetzung neigende Stoffe ausgesprochen ungünstig breites Verweilzeitverhalten vom Rührkesseltyp auf. Daher werden bei der EDC-Verdampfung vor der eigentlichen Pyrolysezone bereits Precursoren genannte Keime für die nachfolgende Rußbildung gebildet.

Dies ist z.B. dadurch belegt, daß bei dem bekannten Verfahren mit spaltgasbeheizter, externer Verdampfung (Wärmerückgewinnung), wie es in den von der Patentanmelderin errichteten Anlagen zur Erzeugung von EDC/VDM zur Anwendung kommt, kontinuierlich ein Abschlämmstrom abgezogen werden muß, um eine vorzeitige Verschmutzung des Verdampfers und der Pyrolyserohre zu vermeiden. Der Abschlämmstrom enthält kohlenstoffreiche, bereits zu größeren Einheiten zusammengewachsene Zersetzungsprodukte.

Aufgabe der Erfindung ist die Schaffung einer Lösung, mit der die Aufheizung des EDC's aus flüssigem Zustand mit Temperaturen unter 100°C auf die Eintrittsbedingungen der Pyrolyse-Zone ohne Verdampfungszone möglich gemacht wird bei gleichzeitiger Ausnutzung einer sinnvollen Wärmerückgewinnung.

Mit einem Verfahren der eingangs bezeichnten Art wird diese Aufgabe gemäß der Erfindung dadurch gelöst, daß das flüssig vorliegende EDC zunächst auf einen Druck über dem kritischen Druck des EDC's (5,36 MPa) gebracht und nachfolgend mindestens auf die kritische Temperatur des EDC's (ca. 288°C) aufgewärmt wird.

Mit der Erfindung ist es möglich, auf den Durchgang durch eine Verdampfungszone verzichten zu können mit der Folge, daß die erwähnten Precursoren für die Rußbildung vermieden werden. Dabei macht sich die Erfindung die Erkenntnis zunutze, daß die Kurve für die isenthalpe Zustandsänderung in einem Druck-Temperatur-Diagramm in der Nähe des kritischen Punktes eine vergleichsweise starke Krümmung aufweist. Dabei ist es dann auch möglich, wie dies die Erfindung in Ausgestaltung vorsieht, den erwärmten EDC-Einsatz zwischenzuentspannen und wieder aufzuheizen, wobei insgesamt nachfolgend das EDC auf die Eintrittsbedingungen der Pyrolyse entspannt wird, die das EDC gasförmig vorfinden bei Temperaturen über 200°C.

Erfindungsgemäß wird der Wärmeinhalt des die Pyrolyse-Zone verlassenden Spaltgases zur Aufheizung des überkritischen Fluids genutzt, wobei dies erfindungsgemäß im einfachen Gegenstromwärmetauscher vorgenommen werden kann.

Hier liegt ein weiterer Vorteil der Erfindung wegen der günstigen Verweilzeiten im Gegenstromwärmetauscher, da auf beiden Seiten ein dem Strömungsrohrtyp entsprechendes Verweilzeitverhalten möglich gemacht wird und damit der oben angesprochenen schädlichen Ruß-/Precursorenbildung weitestgehend entgegengewirkt werden kann, was insgesamt die Rußbildung deutlich reduziert.

Der einzigen Figur kann in vereinfachter Darstellung die 1,2-EDC-Verdampfungskurve bis zum kritischen Punkt entnommen werden, wobei die kurzgestrichelte Linie das Verfahren nach EP-0 276 775-B1 andeutet, die langgestrichelte Linie die erfindungsgemäße Verfahrensweise.

Mit Fig. 1 wird ein Zustandsdiagramm für den reinen Stoff EDC angegeben. Auf der Abszisse des Zustandsdiagramms ist die Temperatur T aufgetragen, auf der Ordinate ist der Druck p aufgetragen. In diesem p,T-Diagramm gibt 1 die Dampfdruckkurve des EDC's wieder. Die Dampfdruckkurve 1 endet im kritischen Punkt 2 des EDC's. Im oberhalb der Dampfdruckkurve liegenden Gebiet sind die Zustände p,T angegeben, bei denen das EDC flüssig vorliegt, unterhalb der Dampfdruckkurve 1 kommt das EDC gas-/dampfförmig vor. Liegen EDC-Zustände auf der Dampfdruckkurve 1, so koexistieren die Dampf- und die Flüssigphase. Nehmen beide EDC-Zustandsgrößen, - also sowohl der Druck als auch die Temperatur-, Werte oberhalb der kritischen Werte (P_{crit} = 53,6 bar, t_{crit} ≈ 288°C) an, so liegt das EDC in Form eines sogenannten überkritischen Fluids vor. Ein überkritisches Fluid besitzt eine in der Größenordnung vergleichsweise ebenso hohe Dichte wie die Flüssigphase und kann jedoch, - anders als es bei der Flüssigphase geschieht -, keine Phasengrenzfläche/ Oberfläche ausbilden.

Im Zustandsdiagramm scheidet die Ordinate P_{crit} das Flüssigkeitsgebiet 5 vom Gebiet des bei überkritischem Druck vorliegenden EDC-Fluids 3. Im ähnlichen Sinne scheidet die Abszisse t_{crit} das Gebiet des überhitzten EDC-Dampfes 4 vom Zustandsgebiet des bei überkritischer Temperatur vorliegenden EDC-Fluids 6.

Der Linienzug A-B1-C1 beschreibt im Zustandsdiagramm die bekannte Erzeugung von überhitztem EDC-Dampf nach EP-0 276 775-B1. Zwischen A und B1 wird der Druck der EDC-Flüssigkeit auf den Verdampfungsdruck gebracht. Von B1 nach C1 erfolgt die Vorwärmung der EDC-Flüssigkeit auf Siedetemperatur, weiterhin die EDC-Verdampfung im Schnittpunkt mit der Dampfdruckkurve 1 und endlich geschieht die EDC-Überhitzung zwischen Dampfdruckkurve und C1.

Die erfindungsgemäße Erzeugung von überkritischem EDC-Fluid dahingegen erfolgt entlang des Linienzuges A-B2-C2. Zwischen A und B2 wird der Druck der EDC-Flüssigkeit auf überkritischen Druck gebracht. Von B2 nach C2 wird bei überkritischem Druck das EDC-Fluid auf eine überkritische Temperatur erhitzt und so das sowohl bezüglich der Temperatur wie des Druckes überkritische EDC-Fluid gebildet.

Die Erhitzung geschieht frei von Phasenumwandlungen, deshalb ändern sich die Transportgrößen des EDC bei dieser Art Zustandsänderung stetig, aber nicht sprunghaft. Z.B. bleibt die Lösefähigkeit des EDC's für die verunreinigenden Begleitstoffe im wesentlichen erhalten und die Konzentrationen der Begleitstoffe besitzen im fluiden EDC die gleichen Werte wie im flüssigen Eingangs-EDC. Auf diese Weise wird die unerwünschte Konzentrationsanreicherung von Begleitstoff-Komponenten des EDC's von vornherein vermieden, wohingegen bei der Bildung von EDC-Dampf aus einer Flüssigphasenoberfläche heraus es immer zu Anreicherungen einzelner Begleitstoff-Komponenten im nicht verdampften Teil der Flüssigkeit kommt. Dies ist ein weiterer Vorzug des erfindungsgemäßen Verfahrens.

Soll das erfindungsgemäß erhitzte EDC vom fluiden Zustand in einen Ausgangszustand versetzt werden, der in etwa dem Ausgangszustand des auf der bekannten Weise mittels Verdampfung erzeugten überhitzten EDC-Dampfes entspricht, so durchläuft das fluide EDC zusätzlich entlang der Linie C2-D-E-F eine zweifache Drosselung mit zwischenliegender Zwischenerhitzung D-E.

## Patentansprüche

1. Verfahren zur Verdampfung von 1,2-Dichlorethan (EDC) vor der thermischen Zersetzung (Pyrolyse),
**dadurch gekennzeichnet,**
**daß** das flüssig vorliegende EDC zunächst auf einen Druck über dem kritischen Druck des EDC's (5,36 MPa) gebracht und nachfolgend mindestens auf die kritische Temperatur des EDC's (ca. 288°C) aufgewärmt wird.

2. Verfahren nach Anspruch 1,
**dadurch gekennzeichnet,**
**daß** das mindestens auf die kritische Temperatur erwärmte EDC zwischenentspannt und wieder aufgeheizt wird.

3. Verfahren nach Anspruch 1 oder 2,
**dadurch gekennzeichnet,**
**daß** das EDC auf die Eintrittsbedingungen der Pyrolyse entspannt wird, mit Temperaturen von 150°C bis 400°C und Drücken von 1 bis 10 MPa.

4. Verfahren nach einem der vorangehenden Ansprüche,
**dadurch gekennzeichnet,**
**daß** der Wärmeinhalt des aus der Pyrolyse-Zone austretenden Spaltgases zur Aufheizung des überkritischen Fluids eingesetzt wird.

5. Verfahren nach einem der vorangehenden Ansprüche,
**dadurch gekennzeichnet,**
**daß** zur Aufheizung des überkritischen EDC's ein Gegenstromwärmetauscher eingesetzt wird.

## Claims

1. Method for evaporating 1,2-dichloroethane (EDC) prior to thermal decomposition (pyrolysis),
**characterised in that**
the EDC which is present in liquid form is first of all brought to a pressure above the critical pressure of the EDC (5.36 MPa) and is subsequently heated to at least the critical temperature of the EDC (approx. 288°C).

2. Method in accordance with claim 1,
**characterised in that**
the EDC which has been warmed up to at least the critical temperature is intermediately relaxed and heated up again.

3. Method in accordance with claim 1 or 2,
**characterised in that**
the EDC is relaxed to the starting conditions of the pyrolysis, with temperatures from 150°C to 400°C and pressures from 1 to 10 MPa.

4. Method in accordance with one of the preceding claims,
**characterised in that**
the caloric content of the cracked gas emerging from the pyrolysis zone is used for heating up the supercritical fluid.

5. Method in accordance with one of the preceding claims,
**characterised in that**
a counterflow heat exchanger is used to heat up the supercritical EDC.

## Revendications

1. Procédé destiné à l'évaporation du dichloro-1,2 éthane (DCE) avant la décomposition thermique (pyrolyse),
**caractérisé en ce que**
le DCE présent sous forme liquide est d'abord porté à une pression supérieure à la pression critique du DCE (5,36 MPa) puis est chauffé au moins jusqu'à la température critique du DCE (environ 288 °C).

2. Procédé selon la revendication 1
**caractérisé en ce que**
le DCE chauffé au moins à la température critique est détendu dans l'intervalle et de nouveau chauffé.

3. Procédé selon la revendication 1 ou 2
**caractérisé en ce que**
le DCE est détendu pour atteindre les conditions d'entrée de la pyrolyse avec des températures comprises entre 150°C et 400°C et des pressions comprises entre 1 et 10 MPa.

4. Procédé selon une des revendications précédentes
**caractérisé en ce que**
la capacité thermique du gaz de craquage sortant de la zone de pyrolyse est utilisée pour chauffer le fluide supercritique.

5. Procédé selon une des revendications précédentes
**caractérisé en ce que**
l'on utilise un échangeur thermique à contrecourant pour chauffer le DCE supercritique.
